# EUROPÄISCHE PATENTANMELDUNG

(11) **EP 2 394 606 A1**
(43) Veröffentlichungstag der Anmeldung: **14.12.2011**
(21) Anmeldenummer: 10006098.7
(22) Anmeldetag: 11.06.2010
(51) Int. Cl.: A61F 2/28, A61F 2/30, A61F 2/36, A61F 2/38

(54) **Prothese zum teilweisen Ersatz eines Röhrenknochens**

(71) Anmelder: WALDEMAR LINK GmbH & Co. KG, 22339 Hamburg (DE)
(72) Erfinder: Link, Helmut D., 22397 Hamburg (DE)
(74) Vertreter: Glawe, Delfs, Moll

(57) **Zusammenfassung**

Die Erfindung betrifft eine Prothese zum mindestens teilweisen Ersatz eines Röhrenknochens und eines angrenzenden Gelenks, umfassend einen länglich gestreckten Stab (1) mit einem ersten und einem zweiten Ende, sowie eine Gelenkeinrichtung (2), die am zweiten Ende des Stabs (1) angeordnet ist, wobei eine Längeneinstellrichtung (3) vorgesehen ist, welche den Stab (1) längs seiner Achse (10) teleskopartig betätigt. Der Stab (1) und die Gelenkeinrichtung (2) sind über eine Steckverbindung mit komplementären Konusverbindern (18,29) gekoppelt, wobei die Längeneinstelleinrichtung (3) modular ausgeführt ist und an ihrem proximalen und distalen Ende mit den komplementären Konusverbindern (18,19) versehen ist, und sie weiter mit einer formschlüssig wirkenden Verdrehsicherung (35,37) versehen ist.

## Beschreibung

Die Erfindung betrifft eine Prothese zum mindestens teilweisen Ersatz eines Röhrenknochens und eines angrenzenden Gelenks. Sie umfasst einen länglich gestreckten Stab mit einem ersten und einem zweiten Ende, sowie eine Gelenkeinrichtung, die am zweiten Ende des Stabs angeordnet ist. Es ist eine Längeneinstellrichtung vorgesehen, welche den Stab längs seiner Achse teleskopartig verschiebt.

Zum Ersatz von erkrankten oder defekten Knochen und Gelenken sind verschiedene Typen von Endoprothesen seit langem bekannt. Zum Ersatz von Röhrenknochen, insbesondere aufgrund von Tumorerkrankungen, werden Prothesen verwendet, die einen sich über die Länge des zu ersetzenden Knochens ausgedehnten Schaft aufweisen. Der Schaft ersetzt bzw. verstärkt den erkrankten oder fehlenden Bereich des Knochens. Häufig ist er verbunden mit einer Gelenkeinheit, welche ein angrenzendes Gelenk (zum Beispiel Knie oder Ellenbogen) ersetzt. Die Abmessungen des Prothesenschafts müssen daher entsprechend der jeweiligen Anatomie und Pathologie der Patienten gewählt sein.

Zur Anpassung an die individuellen Bedürfnisse ist es bekannt, die Prothesen in verschiedenen Größen anzubieten. Jedoch kann selbst mit einer feinen Abstufung keine optimale Versorgung in Anbetracht der Vielzahl an verschiedenen Bedürfnissen erreicht werden. Dies gilt noch viel mehr bei Patienten, die sich in der Wachstumsphase befinden, also Kindern.

Um auch bei solchen Patienten eine ausreichende Versorgung zu ermöglichen, sind Prothesen mit einer Längeneinstelleinrichtung versehen worden. So ist eine Knieprothese bekannt, die einen Schaft und eine Gelenkeinrichtung umfasst, wobei im Schaft eine Teleskopeinrichtung zur Veränderung der Schaftlänge vorgesehen ist (US 4,384,373). Hierbei ist eine Längeneinstellung des Schafts im Rahmen der Operation ermöglicht. Eine nachträgliche Verstellung ist nicht vorgesehen.

Um auch post-operativ die Länge des Schafts einstellen zu können, ist eine weiterentwickelte Prothese bekannt (US 4,502,160), bei der zur Betätigung eine Überwurfmutter vorgesehen ist. Sie weist einen Außenzahnkranz auf, der über einen von lateral anzusetzenden Steckschlüssel mit einer Außenverzahnung betätigbar ist. Der Steckschlüssel kann durch eine Inzision geführt sein, so dass auch nach der Operation die Länge des Schafts verstellt werden kann.

Um auch bei der Veränderung der Länge eine Rotation der Prothese, insbesondere eine Rotation von Schaft relativ zum Gelenk, zu vermeiden, kann eine Rotationssperre vorgesehen sein (US 4,892,546). Mittels einer Sperrschraube ist der Schaft an einer Verdrehung gegenüber dem Gelenk gehindert, wobei zur Längenverstellung die Sperrschraube gelöst ist.

Ein Nachteil dieser bekannten Prothesen liegt darin, dass sie jeweils sehr spezialisiert (post-operativ betätigbar, rotationsgeschützt etc.) sind, und deshalb jeweils nur ein schmales Anwendungsfeld haben.

Der Erfindung liegt die Aufgabe zugrunde, eine Endoprothese der eingangs genannten Art dahingehend weiterzuentwickeln, dass sie universeller einsetzbar ist.

Die erfindungsgemäße Lösung liegt in den Merkmalen der unabhängigen Ansprüche. Vorteilhafte Weiterbildungen sind Gegenstand der abhängigen Ansprüche.

Bei einer Prothese zum mindestens teilweisen Ersatz eines Röhrenknochens und eines angrenzenden Gelenks, umfassend einen länglich gestreckten Stab mit einem ersten und einem zweiten Ende, eine Gelenkeinrichtung, die am zweiten Ende des Stabs angeordnet ist, wobei eine Längeneinstellrichtung vorgesehen ist, welche den Stab längs seiner Achse teleskopartig betätigt, ist erfindungsgemäß vorgesehen, dass der Stab und die Gelenkeinrichtung über eine Steckverbindung mit komplementären Konusverbindern gekoppelt sind, wobei die Längeneinstelleinrichtung modular ausgeführt ist und an ihrem proximalen und distalen Ende mit den komplementären Konusverbindern versehen ist, und sie weiter mit einer formschlüssig wirkenden Verdrehsicherung versehen ist.

Kern der Erfindung ist der Gedanke, die Längeneinstelleinrichtung modular auszugestalten und sie dazu an ihrem proximalen und distalen Ende mit genau solchen Konussteckverbindern zu versehen, die auch am Übergang zwischen Schaft und Gelenk der Prothese angeordnet sind. Die Längeneinstelleinrichtung ist also anders als im Stand der Technik nicht integraler Bestandteil der Prothese, sondern kann nach Bedarf eingesetzt sein. Sie ist sozusagen austauschbar gegen ein herkömmliches Standard-Schaftelement ohne Längeneinstelleinrichtung.

Die Erfindung ermöglicht es damit auf einfache und effiziente Weise, an sich beliebige Gelenkprothesen mit einer Längeneinstelleinrichtung zu versehen. Die Anpassbarkeit der Prothese an die anatomischen bzw. pathologischen Verhältnisse des einzelnen Patienten verbessert sich damit erheblich, und zwar ohne dass dafür eine Vielzahl von verschiedenen Teilen in unterschiedlicher Größe erforderlich wäre. Die Gelenkeinrichtung kann an sich beliebig ausgeführt sein, wobei sie Bewegungen im Gelenk in unterschiedlichem Grad stützen oder beschränken kann von völlig frei bis zu versteift. Indem die Längeneinstelleinrichtung erfindungsgemäß von der eigentlichen Gelenkprothese getrennt ist dank der modularen Ausführung, kann die Erfindung ohne weiteres auch an anderen Prothesen angewendet sein, solange sie nur über entsprechende Konussteckverbinder verfügen. Dank der integrierten formschlüssig wirkenden Verdrehsicherung sind keine weiteren Anforderungen in Bezug auf Verdrehschutz an die jeweilige Grundprothese gestellt.

Die bauliche Integration von Verdrehsicherung und Längeneinstellung hat weiter den Vorteil, dass die Betätigungselemente eng beieinander liegen können. Damit ist zur postoperativen Verstellung nur ein Zugang an einer räumlich eng umgrenzten Stelle erforderlich. Es genügt eine nur minimal invasive Stichinzision, um die Prothese in ihrer Länge zu verändern. Mit einer solch schonenden Operationstechnik ist die Prothese insbesondere auch zur Verwendung bei Kindern geeignet.

Vorzugsweise weist der Schaft einen Außen- und einen Innenstab auf, auf welchen die Längeneinstelleinrichtung wirkt. Damit kann mittels eines geeigneten Werkzeugs unmittelbar auf die Längeneinstelleinrichtung eingewirkt werden, die entsprechend den Außenstab teleskopartig gegenüber dem Innenstab verschiebt.

Um trotz der einfachen Verstellbarkeit einen ausreichenden Schutz vor unbeabsichtigter Verstellung zu gewährleisten, ist vorzugsweise eine Doppelsicherung vorgesehen, welche zusätzlich zur Verdrehsicherung eine Verstellsicherung mittels zweier benachbarter Schrauben bildet. Hierbei ist weiter vorzugsweise die eine drehfest am Außenstab und die andere drehbeweglich an der Verstellmutter angeordnet.

Der Außenstab weist mit Vorteil einen Druckflansch auf, der zwei einander gegenüberliegende Bundflächen aufweist, von denen die eine ein Schublager für die Konusverbindung und die andere einen Anschlag für die Längeneinstellung bildet. Damit ist ein sehr kompakter Aufbau ermöglicht, der eine Integration der erfindungsgemäßen Längeneinstelleinrichtung auch an verhältnismäßig kleine Prothesen ermöglicht, beispielsweise zur Anwendung am Ellbogen oder an der Hand.

Es kann zweckmäßig sein, wenn eine zweite Längeneinstelleinrichtung für den Schaft vorgesehen ist, die vorzugsweise mit invers angeordneten Konusverbindern versehen ist. Bei langen Schäften, insbesondere solchen zum Ersatz des Femurs, kann damit auch am anderen Ende eine Längeneinstellung erfolgen. Dies vergrößert nicht nur den Einstellbereich, sondern ist häufig auch physiologisch günstiger.

Die Erfindung erstreckt sich weiter auf ein Prothesensystem mit mehreren koppelbaren verschieden langen starren Schaftelementen, und einer koppelbaren Längeneinstelleinrichtung, die vorzugsweise in ihrer Grundstellung dieselbe Länge wie eines der starren Schaftelemente aufweist. Damit können innerhalb eines Prothesensystems solche Prothesen gebildet sein, die einen Schaft mit starrer Länge oder einen Schaft mit einstellbarer Länge aufweisen, wobei durch einfachen Austausch eines starren Schaftmoduls gegen eines mit einstellbarer Länge hin- und hergewechselt werden kann. Dies kann auch intraoperativ erfolgen, so dass je nach Lage des Falls der Chirurg noch im Laufe der Operation entscheiden kann, welche Variante im jeweiligen Fall vorzugsweise verwendet werden soll.

Die Erfindung wird nachfolgend unter Bezugnahme auf die beigefügte Zeichnung erläutert, in der vorteilhafte Ausführungsbeispiele dargestellt sind. Es zeigen:
- Fig. 1: eine Schnittansicht für eine Kniegelenkprothese gemäß einem ersten Ausführungsbeispiel der Erfindung;
- Fig. 2: eine Totalprothese auf Grundlage des ersten Ausführungsbeispiels gemäß Fig. 1 in Frontal und Lateralansicht;
- Fig. 3: Explosionsansichten zu Fig. 2;
- Fig. 4: eine Schnittansicht zu einem zweiten Ausführungsbeispiel der Erfindung; und
- Fig. 5a-e: Darstellungen zur Durchführung einer Längenverstellung.

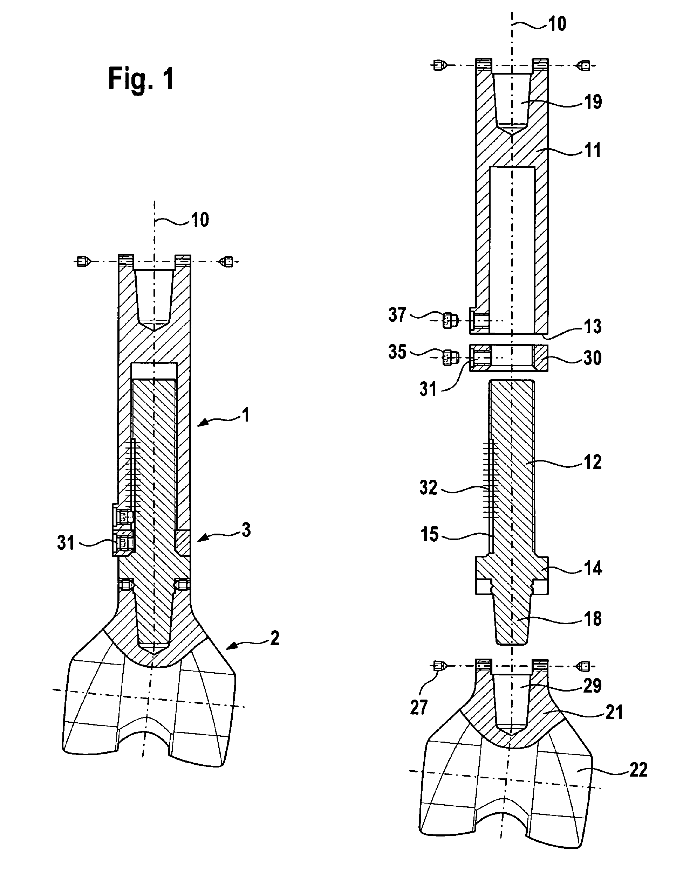

In Fig. 1 ist ein Ausführungsbeispiel der erfindungsgemäßen Prothese dargestellt, das als Gelenkprothese zum teilweisen Ersatz des Knies und eines Teils des distalen Femurs vorgesehen ist. Sie umfasst als Komponenten einen Schaft 1, eine Gelenkeinrichtung 2 und eine Längeneinstelleinrichtung 3.

Der Stab 1 umfasst einen Außenstab 11 und einen Innenstab 12, der teleskopartig längs seiner Mittelachse 10 verschieblich in dem Außenstab 11 geführt ist. Der Außenstab 12 weist an seinem ersten Ende einen weiblichen Konussteckverbinder 19 auf, der bei Bedarf zum Ankoppeln weiterer Stabsegmente (in Fig. 1 nicht dargestellt) dient; es sei angemerkt, dass sie auch durch einen Blindstopfen verschlossen sein kann oder ganz entfallen kann. An seinem zweiten Ende weist der Außenstäb einen Stirnflansch 13 mit einer radial ausgerichteten Stirnfläche auf. Der Innenstab 12 weist an seinem ersten Ende einen männlichen Konussteckverbinder 18 auf, der zum Eingriff in einen passenden weiblichen Konussteckverbinder 29 an der Gelenkeinrichtung 2 ausgebildet ist. Am Übergang zu dem Konussteckverbinder 18 weist der Innenstab 12 einen Bund 14 auf, dessen eine, dem festen Ende zugewandte Stirnfläche als Anschlag für den Konussteckverbinder 18 und dessen andere, dem Schaft des Innenstabs 12 zugewandte Stirnfläche als Anschlag für eine Verstellmutter 30 fungiert.

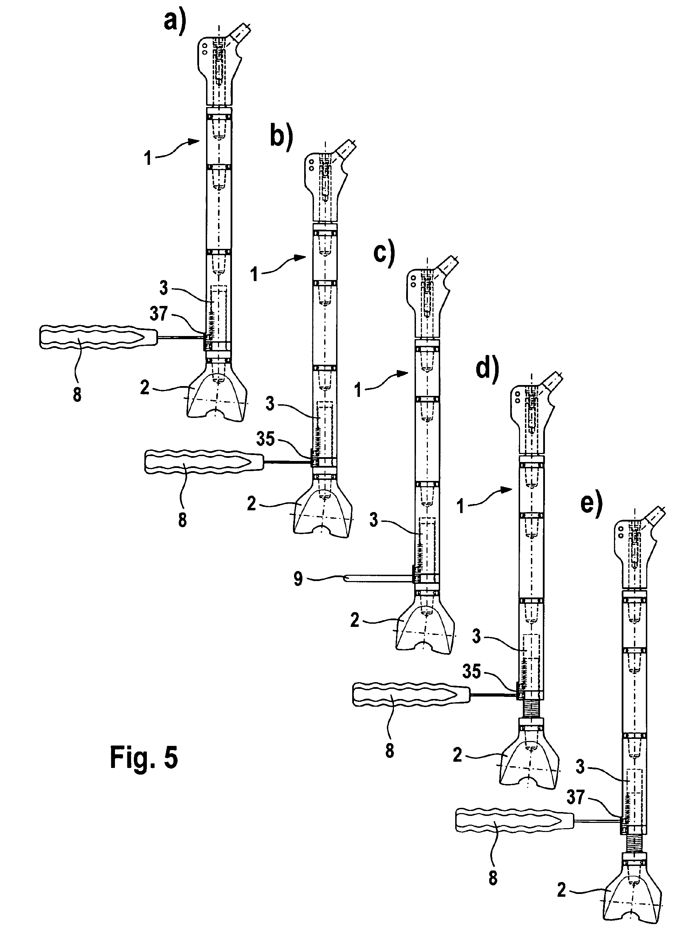

Die Betätigungseinrichtung 3 umfasst die Verstellmutter 30 mit einem Innengewinde, das mit einem auf dem Innenstab 12 angeordneten Stellgewinde 32 kämmt. Die Verstellmutter 30 weist an ihren Seitenflächen eine Mehrzahl von Eingriffsöffnungen 31 auf, die als Radialbohrungen ausgestaltet sind. Sie sind dazu ausgebildet, einen Stift 9 (s. Fig. 5c) als Betätigungselement aufzunehmen. Damit wird die Verstellmutter 30 gegenüber dem Innenstab 12 mit dem Gegengewinde 32 verdreht, wodurch die Verstellmutter 30 entlang der Mittelachse 10 sich bewegt. Die Verstellmutter 30 liegt in Grundstellung unmittelbar an dem Stirnflansch 13 des Außenstabs 11 an und nimmt diesen bei ihrer Bewegung mit. Dadurch bewegt sich der Außenstab 11 entlang der Längsachse 10 relativ zu dem Innenstab 12 derart, dass sich der Abstand zwischen Verstellmutter 30 und dem Bund 14 vergrößert und die Gesamtlänge des Schafts 1 zunimmt. Bei Verdrehung der Verstellmutter 30 in Gegenrichtung läuft der Vorgang in umgekehrter Richtung ab und die Gesamtlänge verkürzt sich.

Zur Fixierung einer eingestellten Länge sind Sicherungseinrichtungen vorgesehen. Sie umfassen eine Verstellsicherung 35 und eine Verdrehsicherung 37. Die Verstellsicherung 35 weist eine Klemmschraube auf, die in eine der radialen Bohrungen 31 der Verstellmutter 30 eingesetzt ist und mit ihrer Spitze auf eine Abflachung 15 am Außenstab 12 einwirkt. Damit wird eine formschlüssige Verriegelung der Verstellmutter 30 erreicht. Es besteht so Sicherheit, dass auch bei großer Last und häufigen Lastwechseln keine unbeabsichtigte Verdrehung der Verstellmutter 30 mit entsprechender Längenänderung entstehen kann. Die Verdrehsicherung 37 ist ähnlich aufgebaut und weist eine Fixierschraube auf, die in eine Radialbohrung im Bereich des Stirnflansch 13 am Außenstab angeordnet ist. Die Fixierschraube wirkt mit ihrer Spitze in den Bereich des Gegengewindes 32 und sichert damit den Außenstab 11 vor einer Verdrehung gegenüber dem Innenstab 12. Dieser ist wiederum über eine an sich bekannte Konussicherung 27 mittels zweier diametral gegenüberliegender Fixierschrauben an einer unerwünschten Verdrehung gegenüber der Gelenkeinrichtung 2 gehindert. Damit ergibt sich eine durchgehende Rotationssicherung von der Gelenkeinrichtung 2 über die Längenverstelleinrichtung 3 bis zum Schaft 1.

Der Vorgang der Längenverstellung ist in Fig. 5 verdeutlicht am Beispiel einer implantierten Prothese, die zur Anpassung an das Wachstum des Patienten auf eine größere Länge eingestellt werden soll. Dazu ist es zuerst erforderlich, mittels minimal invasiver Chirurgie einen Zugang zur Prothese zu schaffen. In der Regel genügt hierfür eine Stichinzision. In einem ersten Schritt (Fig. 5a) wird ein Schraubendreher 8 durch die Inzision geführt und in Eingriff mit der Sicherungsschraube für die Rotationssicherung 37 gebracht. Die Rotationssicherung wird durch Herausdrehen der Schraube gelöst. In einem zweiten Schritt (Fig. 5b) wird die Verstellsicherung 35 auf dieselbe Weise gelöst. Damit ist die Längenverstelleinrichtung 3 frei und kann betätigt werden. Der Schraubendreher 8 wird entfernt und ein Verstellstift 9 durch die Inzision eingeführt und in Eingriff mit einer der Radialbohrungen 31 der Verstellmutter gebracht. Durch Verschwenken des Stifts 9 wird die Verstellmutter 30 ein Stück gedreht, und der Stift 9 in eine benachbarte Radialbohrung eingesetzt und die Verstellmutter 30 wieder ein Stück gedreht. Bei dem dargestellten Ausführungsbeispiel ist die Gewindesteigung so gewählt, dass sich pro Umdrehung der Verstellmutter 30 eine Längenänderung um 2 mm ergibt. Ist die gewünschte Länge eingestellt, wird der Stift 9 entnommen und wiederum der Schraubendreher 8 eingeführt, um nacheinander die Muttersicherung 35 (Fig. 5d) und die Rotationssicherung (Fig. 5e) wieder einzusetzen und damit zu sichern.

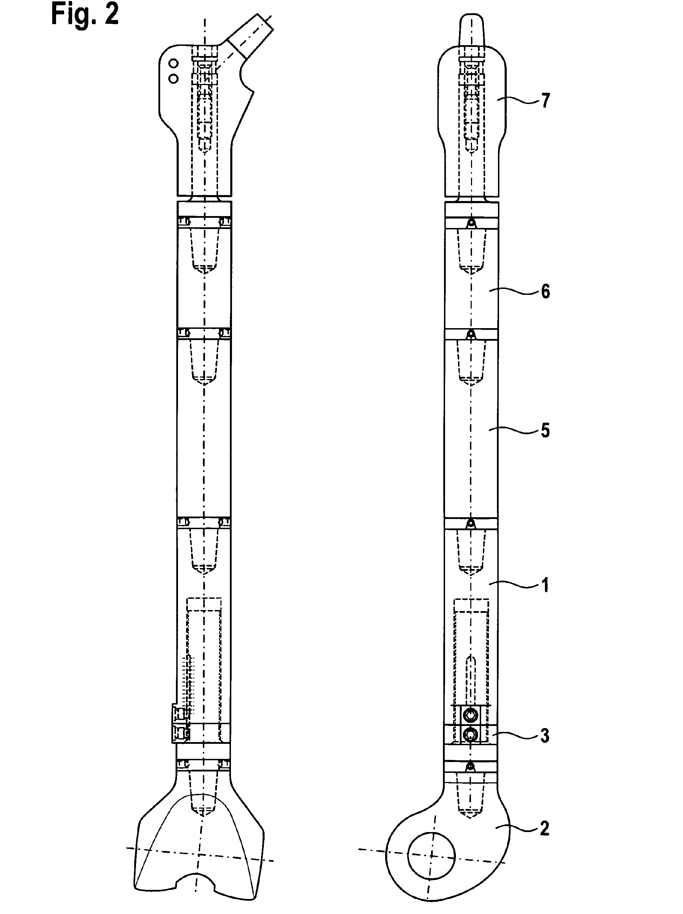

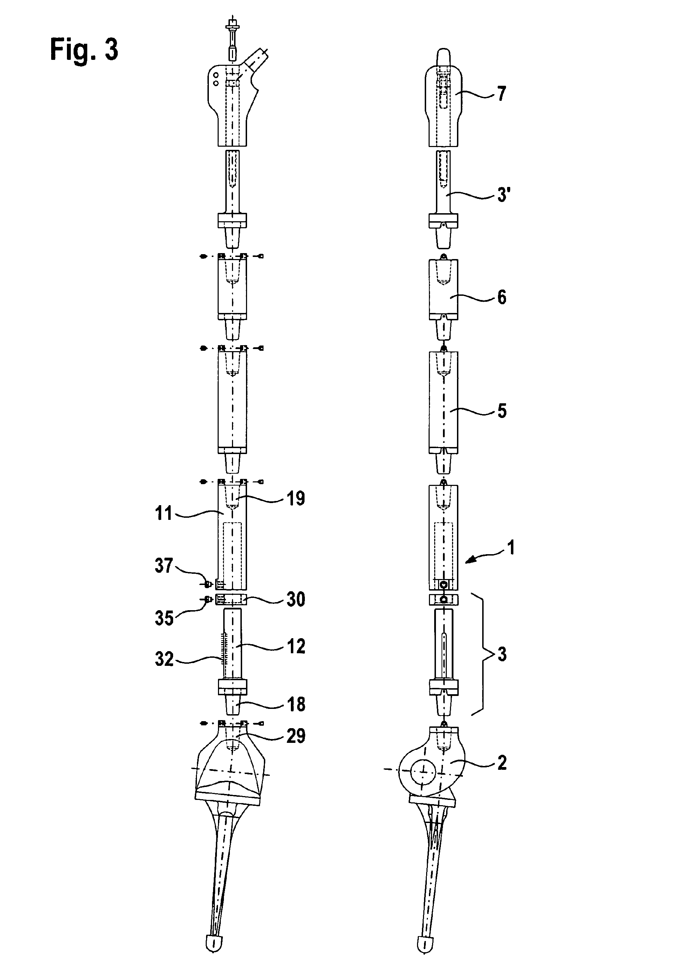

Bei der in Fig. 1 dargestellten Ausführungsform handelt es sich um eine Basis-Prothese. Sie kann mit zusätzlichen Elementen ergänzt werden, wie in den Fig. 2 und 3 dargestellt. Dort sind zusätzliche Schaftsegmente 5, 6 vorgesehen, die über Konussteckverbinder, die passend sind zu den Konussteckverbindern 18, 19 des Schafts 1 und 29 der Gelenkeinrichtung 2, zusammengesetzt sind zu einem langen Schaft (s. Explosionsdarstellung in Fig. 3). An dessen oberen Ende ist eine Femurhalsprothese angeordnet. Damit ist eine Femurtotalprothese gebildet, die nicht nur wie im Stand der Technik durch Auswahl passender Schaftsegmente 5, 6 mit abgestufter Länge gebildet sein kann, sondern dank der modularen Längeneinstelleinrichtung 3 sogar stufenlos verstellbar ist. Damit kann eine Feinanpassung vorgenommen werden. Vorzugsweise hat der Schaft 1 mit der Längenverstelleinrichtung 3 in einer Grundstellung (wie in Fig. 1 dargestellt) dieselbe Länge wie eines der Schaftsegmente, zum Beispiel das Schaftsegment 5. Damit ist ein Prothesensystem gebildet, bei dem je nach Bedarf ein längeneinstellbarer oder ein längenfester Schaft durch einfachen Austausch der Elemente 1, 5 gebildet werden kann.

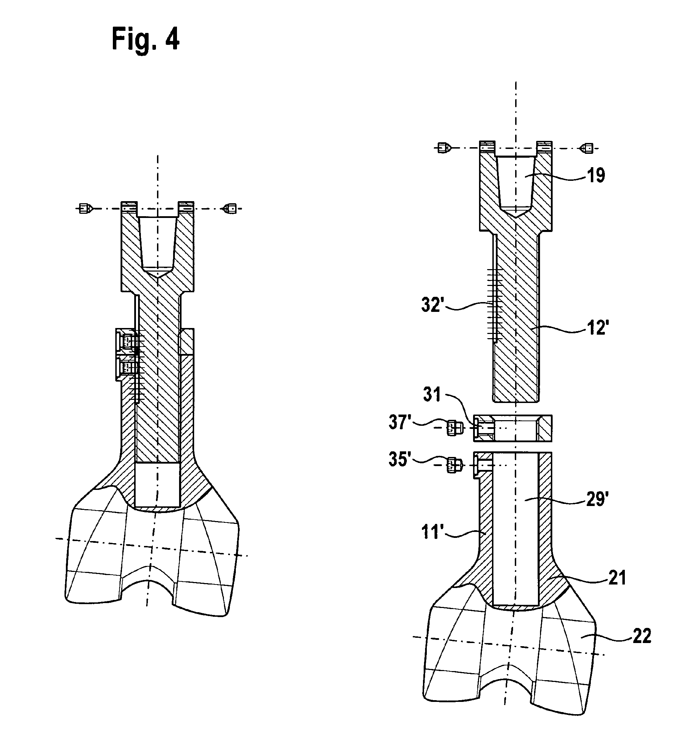

Ein alternatives Ausführungsbeispiel ist in Fig. 4 dargestellt. Mit dem in Fig. 1 dargestellten Ausführungsbeispiel sind identische Elemente mit denselben Bezugsziffern versehen. Der Unterschied zu dem ersten Ausführungsbeispiel besteht im Wesentlichen darin, dass die Anordnung des Außenstabs 11' und Innenstabs 12' invers ausgeführt ist, d. h. der Außenstab 11' ist an der Gelenkeinrichtung 2 angeordnet und der Innenstab 12' bildet das erste Ende mit der Konussteckverbindung 19. Eine solche invers ausgeführte Längeneinstelleinrichtung 3' kann auch am ersten Ende eines langen Schafts mit mehreren Schaftsegmenten 5, 6 vorgesehen sein, wie in Fig. 3 dargestellt.

## Patentansprüche

1. Prothese zum mindestens teilweisen Ersatz eines Röhrenknochens und eines angrenzenden Gelenks, umfassend einen länglich gestreckten Stab (1) mit einem ersten und einem zweiten Ende, sowie
eine Gelenkeinrichtung (2), die am zweiten Ende des Stabs (1) angeordnet ist,
wobei eine Längeneinstellrichtung (3) vorgesehen ist, welche den Stab (1) längs seiner Achse (10) teleskopartig betätigt, **dadurch gekennzeichnet, dass** der Stab (1) und die Gelenkeinrichtung (2) über eine Steckverbindung mit komplementären Konusverbindern (18, 29) gekoppelt sind, wobei die Längeneinstelleinrichtung (3) modular ausgeführt ist und an ihrem proximalen und distalen Ende mit den komplementären Konusverbindern (18, 19) versehen ist, und sie weiter mit einer formschlüssig wirkenden Verdrehsicherung (35, 37) versehen ist.

2. Prothese nach Anspruch 1, **dadurch gekennzeichnet, dass** der Schaft (1) einen Innenstab (12) und einen koaxialen Außenstab (11) umfasst, auf welche die Längeneinstelleinrichtung (3) einwirkt, und die Verdrehsicherung (37) am Außenstab (11) angeordnet ist und in eine Längsausnehmung (15) am Innenstab (12) eingreift.

3. Prothese nach Anspruch 1 oder 2, **dadurch gekennzeichnet, dass** eine Doppelsicherung vorgesehen ist, welche zusätzlich zur Verdrehsicherung (37) eine Verstellsicherung (35) umfasst.

4. Prothese nach Anspruch 3, **dadurch gekennzeichnet, dass** die Verdrehsicherung (37) drehfest und die Verstellsicherung (35) drehbeweglich angeordnet sind.

5. Prothese nach einem der vorangehenden Ansprüche, **dadurch gekennzeichnet, dass** ein Druckflansch (15) vorgesehen ist, der zwei einander gegenüberliegende Bundflächen aufweist, von denen die eine ein Schublager für die Konusverbinder und die andere einen Anschlag für die Längenverstellung bildet.

6. Prothese nach einem der vorangehenden Ansprüche, **dadurch gekennzeichnet, dass** eine zweite Längeneinstelleinrichtung (3') für den Schaft (1) vorgesehen ist.

7. Prothese nach einem der vorangehenden Ansprüche, **dadurch gekennzeichnet, dass** die Längenstelleinrichtung (3) einen in Grundstellung gekapselten Schraubtrieb (30, 32) aufweist.

8. Prothesensystem umfassend eine Prothese nach einem der vorangehenden Ansprüche und mehreren verschieden langen Schaftelementen (5, 6), die über die Konusverbinder koppelbar sind.

9. Prothesensystem nach Anspruch 8, **dadurch gekennzeichnet, dass** die Längeneinstelleinrichtung (3) in ihrer Grundstellung dieselbe Länge wie eines der Schaftelemente (5, 6) aufweist.
